# EUROPEAN PATENT APPLICATION

(11) **EP 3 124 047 A1**
(43) Date of publication of application: **01.02.2017**
(21) Application number: 15178604.3
(22) Date of filing: 28.07.2015
(51) Int. Cl.: A61K 45/06, A61K 31/56, A61P 3/06, A61Q 19/06, A61K 9/00

(54) **PENTACYCLIC TRITERPENOIDS FOR INJECTION LIPOLYSIS**

(71) Applicant: Merz Pharma GmbH & Co. KGaA, 60318 Frankfurt am Main (DE)
(72) Inventor: Abel, Ulrich, 61350 Bad Homburg (DE); Abts, Harry Frank, 61440 Oberursel (DE); Hengl, Thomas, 60437 Frankfurt (DE); Parsons, Christopher Graham Raphael, 61130 Nidderau (DE); Danysz, Wojciech, 61130 Nidderau (DE)
(74) Representative: Jacobi, Markus Alexander

(57) **Abstract**

The present invention relates to a composition comprising a pentacyclic triterpenoid compound or a pharmaceutically acceptable salt thereof, and at least one further component C for use in a method for local treating or preventing a condition associated with an undesired local subcutaneous fat deposit in a mammal, wherein said compound is injected into said local subcutaneous fat deposit aiming for its reduction. Further, the present invention relates to the cosmetic use of such composition.

## Description

The present invention relates to a composition comprising a pentacyclic triterpenoid compound or a pharmaceutically acceptable salt thereof, and at least one further component C for use in a method for local treating or preventing a condition associated with an undesired local subcutaneous fat deposit in a mammal, wherein said compound is injected into said local subcutaneous fat deposit aiming for reduction of local subcutaneous fat. Further, the present invention relates to the cosmetic and aesthetic medicinal use of such composition.

Body contouring by means of injection lipolysis plays an increasing role in a number of pharmaceutical and cosmetic applications. Herein, a composition comprising such agent is directly injected into one or more local subcutaneous fat deposit(s) (depot(s)) of interest. For this purpose, the composition comprising such agent is directly injected into the respective target fat-tissue in the mammal's body *(in situ).* It is thereby intended to reduce such one or more local subcutaneous fat deposit(s) in size. This is typically achieved by a partly or complete degradation of the respective adipose tissue (fat-tissue) by means of a destruction of the adipocytes (fat cells) in the local subcutaneous fat deposit(s) of interest. The released cellular fragments are typically removed by intrinsic, physiologic mechanisms such as by means of phagocytosis by macrophages.

Several natural bile acids such as deoxycholate (DC), chenodeoxycholic acid (CDCA), ursodeoxycholate (UDCA) and lithocholic acid (LCA) are used for cosmetic applications in the art. Additionally, phosphatidylcholine (PC) may be added to compositions used for this purpose. These compounds are described as suitable for reducing adipose tissue by means of injection lipolysis. However, these compounds used in the literature lead to severe undesired side effects and potential toxicity issues. Remarkably, the adipocytolytic effect found when using such compounds is due to the fact that said compounds serve as rather harsh detergents that destroy the cellular membranes of the adipocytes and emulsify the released lipids in a mere and harsh physicochemical way.

Therefore, the compounds used in the art may to a large extend result in a necrotic cell death of the treated adipocytes. This regularly provokes severe side effects such as significant inflammatory activities. This can severely endanger mammal's (in particular human's) health status. Mammal's compliance is often rather low and many individuals refrain from a treatment by injection lipolysis because of the awareness of the expected side effects. Severe and painful local irritations as well as systemic intolerances are frequently observed. In addition, the harsh way of action makes controlling the desired gradual effect difficult to reach.

On the other hand, sugar-conjugates such as glycyrrhizic acid are used (US 2013/0157967), which however are rather complex in structure and not easy to handle.

Therefore, there is an unmet need for compounds and compositions that can be effectively and safely used for injection lipolysis and, concomitantly, offer good controllability of desired effects and cause less severe side effects. In particular for body contouring, i.e., for shaping a mammal's outer appearance (for pharmaceutical and/or cosmetic purposes) mammal's compliance is desired to be significantly improved. Therefore, there is a need for compounds and compositions that bear a high efficiency on the targeted adipocytes, offer good controllability of this effect and are well-tolerably by the mammal's body.

Another disadvantage of the natural bile acids is that they are mostly obtained from animal bodies, in particular mammals' bodies. First, this causes a considerable risk of transmission of pathogens such as viruses and bacteria. Second, concerns of several patients to obtain administrations of natural bile acids may be rather high due to the fact that natural bile acids are mostly obtained from sacrificed animals - may it be for religious or ethical reasons. Therefore, also for these reasons, it is desired to use compounds from other sources.

Surprisingly, it has been found that instead of such bile acids, pentacyclic triterpenoid compounds may be used for injection. The injection into undesired local subcutaneous fat deposits *(in situ)* thereby reduces the fat deposits in size and are well-tolerated by the mammal's body.

In addition to the desired adipocytolytic effect, the pentacyclic triterpenoid compounds bear pharmacological supportive activities (e.g. pro-lipolytic activity and apoptosis). This effect would support their application in body contouring since it increases the desired fat-tissue reduction effect. Exemplarily, ursolic acid is generally known to stimulate the release of stored fat by endogenous lipolysis in adipocytes (Li, 2010, Mol. Nutr. Food Res. 54:1609-1617) and may act against systemic obesity (Kunkel, 2012, PLoS One 7(6):e39332; WO 2014/014530) and hyperlipidemia (Wang, 2013, European Journal of Pharmaceutical Sciences 50:366-371). Further, ursolic acid is generally known to systemically regulate hepatic lipid metabolism (Jia, 2011, Bioorg. Med. Lett. 21:5876-5880). In addition ursolic acid is reported to be able to trigger apoptosis (Shan, 2009, J Zhejiang Univ Sci B 10(9):668-674; Wang, 2011, J Biomed and Biotech, Article ID 419343, doi:10.1155/2011/419343). For elimination of unwanted fat cells, induction of apoptosis would be beneficial since the apoptotic cell death results in much less inflammatory response.

Betulinic acid for instance is known to be able to trigger systemic adipocyte lipolysis (Kim, 2012, Phytother. Res. 26:1103-1106) and cholesterol efflux (Zhao, 2013, PLoS One 8(9):e74782) and to act against systemic obesity (US 2006/0025354). Furthermore, betulinic acid was reported to have pro-apoptotic activity (Schmidt, 1997, European Journal of Cancer 33(12):2007-2010; Fulda, 2000, Medicinal and pediatric Oncology 35:616-618).

The pentacyclic triterpenoid compounds are thus, able to reduce undesired local subcutaneous fat deposits *(in situ)* by means of a combination of physicochemical properties and pharmacologic actions triggering endogenous lipolysis and apoptosis in adipocytes. The physicochemical properties allow good controllability of the desired elimination of adipocytes, while the pharmacological effects support the targeted reduction of undesired fat-tissue. In summary an efficient, controllable elimination of undesired fat-tissue can be reached with significantly reduced side effects. Further, the suggested compounds can be safely purified from plants, thus, avoiding the use of material from animal sources and thus eliminates the risk of animal transmitted diseases.

In a first aspect, the present invention relates to a composition comprising a pentacyclic triterpenoid compound or a pharmaceutically acceptable salt thereof, and at least one further component C for use in a method for local treating or preventing a condition associated with an undesired local subcutaneous fat deposit in a mammal, wherein said compound is injected into said local subcutaneous fat deposit.

The present invention relates to a method for local treating or preventing a condition associated with an undesired local subcutaneous fat deposit in a mammal, wherein a composition comprising a pentacyclic triterpenoid compound or a pharmaceutically acceptable salt thereof and at least one further component C is injected into said local subcutaneous fat deposit *(in situ).* The composition of the present invention results in the elimination of adipocytes by cell death (adipocytolysis). In part the elimination of adipocytes is reached by compound-induced apoptosis. Compound-mediated pharmacological induction of endogenous lipolysis may deliver a further supportive activity: First, the size of non-eliminated adipocytes is reduced by the release of stored fat. Second, the uptake of fat from destroyed fat cells by surrounding fat cells is reduced.

It will be understood that "pentacyclic" means that the compound comprises five rings. A triterpenoid may be understood in the broadest sense as a compound that is structurally derivable from a triterpene, i.e., a terpene obtainable from six isoprene units. The pentacyclic triterpenoid compound is herein also designated by the terms "the pentacyclic triterpenoid compound", "the compound of the present invention", "the compound" and "the compound comprised in the composition of the present invention". The person skilled in the art will understand that these terms are understood interchangeably.

The person skilled in the art will note that the pentacyclic triterpenoid compound of the present invention will typically not be covalently conjugated with one or more sugar moieties (i.e., not be the sugar-conjugates of the respective pentacyclic triterpenoid compounds) such as e.g., glycyrrhizic acid. In particular, at C-atom position 3, the pentacyclic triterpenoid compound of the present invention will typically not be conjugated with a sugar moiety.

Preferably, the pentacyclic triterpenoid compound comprises at least one (unbound) hydroxyl group (-OH), or at least one acetylated hydroxyl group (-O+-CO-CH₃) and/or at least one carbonyl group (C=O) at the A ring of the pentacyclic triterpenoid compound, in particular bears a -OH, -O-CO-CH₃ or an =O group attached to the C-atom in position 3 of the pentacyclic triterpenoid compound.

A pharmaceutically acceptable salt may be understood in the broadest sense as any salt that bears a comparably low toxicity and is acceptable for pharmaceutical purposes. Exemplarily but not limiting, a pharmaceutically acceptable salt may comprise a cation selected from the group consisting of an alkali metal (in particular, Na⁺ and/or K⁺), a proton (i.e., H⁺), an alkaline earth metal (in particular, Mg²⁺ and/or Ca²⁺), ammonium (NH₄⁺), Fe²⁺, Fe³⁺, Zn²⁺, Sn²⁺, and an organic amine cation.

As the composition according to the present invention may optionally also comprise one or more salts, it may also comprise anions like, exemplarily, such selected from the group consisting of a halogen (in particular Cl⁻, Br⁻, I⁻ and/or F⁻), OH⁻, H₂PO₄⁻, HPO₄²⁻, PO₄³⁻, SO₄²⁻, an anion of an organic acid (e.g., acetate, methanoate, propionate, a salt of a fatty acid, gluconate, lactate, citrate, etc.), an organic sulfonate, an organic sulfate, and organic phosphate.

An undesired local subcutaneous fat deposit in a mammal may be understood in the broadest sense as any unwanted accumulation in the mammal's body that bears a considerable content of adipose tissue (fat-tissue), i.e., a tissue comprising adipocytes (fat cells). Preferably, in such tissue, adipocytes constitute for at least 25%, more preferably at least 50%, of the tissue volume. It will however be understood by the person skilled in the art that also the fat deposit will typically comprise other cell types such as cell types forming blood vessels, fibroblasts, blood cells and immune cells. The fat deposit to be treated is located subcutaneously, i.e., beneath the outer skin (deep dermis). The fat deposit of interest is typically located in the subcutis (hypodermis, hypoderm, subdermis).

The subcutaneous fat deposits are normally local fat deposits. Therefore, the present invention focuses on treating or preventing one or more subcutaneous fat deposit(s) in a particular part of the mammal's body rather than on a systemic treatment of disseminated or general obesity.
These specific local subcutaneous fat deposits are often undesired and recalcitrant to diet and exercise. Therefore, it is intended to remove said fat deposit or at least reduce said fat deposit size.

According to the present invention, the pentacyclic triterpenoid compound comprised in the composition of the present invention is directly injected into the undesired local subcutaneous fat deposit *in situ.* Therefore, the composition of the present invention is directly brought in contact with the respective adipose tissue. A first pass effect obtained when administering the composition of the present invention orally is therefore circumvented. Further, systemic side effects observable when administering such composition systemically (e.g, orally, nasally, intravenously (i.v.), intraarterially (i.a.), intraperitoneally (i.p.), intramusculary (i.m.)) are also avoided. Therefore, comparably low systemic side effects, but rather side-specific (i.e., local) effects on the particular undesired subcutaneous fat deposit are observed.

Injection into the undesired local subcutaneous fat deposit may be injection once ((acute) single administration) or may be repeated injection. Repeated injection may exemplarily be injection two times, three times, four times, five times, six times, seven times, eight times, nine times, ten times, or more than ten times. Preferably, injection is made 1 to 6 times. Therefore, the injection may be exemplarily an injection only once, two injections, three injections, four injections, five injections, or six injections.

As used herein, each injection means application within one session, i.e., typically within less than one hour. The person skilled in the art will note that a single injection may optionally include several punctures of a single local fat deposit from different sides and/or angles and/or of more than one local fat deposit within a single session. Each puncture is a penetration of the mammal's skin with an injection tool, such as a needle accompanied by applying the composition of the present invention through said injection tool to the local fat deposit punctured. Exemplarily, each local fat deposit may be punctured once, may be punctured twice, may be punctured 3 times, may be punctured 4 times, may be punctured 5 times, may be punctured 6 times, or may be punctured more than 6 times at different sides and/or from different angles.

Fat deposits may be punctured more than once in a single session. Then, preferably, the distance between two punctures (spacing of the punctures) is preferably at least 0.25 cm, more preferably at least 0.5 cm, in particular at least 1 cm or even more than 2 cm such as at least 3 cm, 4 cm or at least 5 cm. In other words, there are preferably not more than 4 punctures per square centimeter (cm²), more preferably not more than 2 punctures per cm², even more preferably not more than 1 punctures per cm² or even less such as not more than 0.5 or not more than 0.25 or not more than 0.1 punctures per cm². Exemplarily, for treating a double chin, up to 20 single punctures may be used in one session.

Between two injection sessions, there may be a time interval of less than 24 hours, between one and seven days, between one week and two weeks, between two weeks and a month, between a month and six months, between six months and a year. Injective administration may be daily, every second day, every three days, weekly, biweekly, monthly, twice a year, yearly, or less often. Preferably, between two injections, there may be a time interval of 1 to 4 weeks. Exemplarily, the time interval may be in the range of from 5-9 days, 9-16 days, 16-23 days, 23 to 30 days.

More preferably, an undesired fat deposit in a mammal is injected once (a single injection session) or 2 to 6 times (2 to 6 injection sessions) with a time interval between two injections of 1 to 4 weeks. Clinically viable administration schemes may be determined by the person skilled in the art based on balancing efficacy and toxicity. Injection may exemplarily be injection with a syringe.

The volume of the liquid or semi-solid (i.e., viscous) composition of the present invention injected into the mammal per injection may optionally be in the range of several microliters to several milliliters. Exemplarily, the amount injected at once in a single injection may be in the range of 50-150 µl, in the range of 150-300 µl, in the range of 300-500 µl, in the range of 400-600 µl, in the range of 500-1000 µl or in the range of 1-2 ml. The person skilled in the art will notice that the volume of the liquid or semi-liquid composition injected into the mammal per injection will be adjusted to the size of the fat deposit and the concentration of the active compound in the composition.

As used throughout the present invention, the term "mammal" may be understood in the broadest sense as any mammalian animal including humans, irrespective whether clinical symptoms occur or do not occur. Preferably, the mammal is a human or a domestic animal such as an animal selected from the group consisting of mouse, rat, cow, pig, dog, cat, and horse. Most preferably, the mammal is a human (e.g. patient).

Particularly preferably, the composition of the present invention is a pharmaceutical composition.

The at least one further component C may be any component that is pharmaceutically acceptable, in particular pharmaceutically acceptable for being injected into a local fat deposit. Exemplarily, the at least one further component C may be selected from one or more pharmaceutically acceptable carrier(s), one or more detergent(s) and one or more pharmaceutically acceptable additive(s). Such components are further exemplified below.

The person skilled in the art will adjust the dose injected in a single shot in view of the size of the undesired subcutaneous fat deposit to be treated or prevented, the body weight and health status of the mammal and the administration scheme chosen.

In a preferred embodiment, injecting said composition into the local subcutaneous fat deposit is for reducing the size of said local subcutaneous fat deposit, in particular for body contouring.

Reducing in size may be understood in the broadest sense as any decrease of the volume of the undesired local subcutaneous fat deposit. Reducing in size may be a decrease of the volume of the undesired local subcutaneous fat deposit in which the pentacyclic triterpenoid compound comprised in the composition of the present invention is injected by at least 5 %, at least 10 %, at least 25 %, at least 50 %, at least 75 %, at least 90 %, or at least 95 % in volume.

A large variety of conditions associated with an undesired local subcutaneous fat deposit are known in the art.

In a preferred embodiment, the condition associated with an undesired local subcutaneous fat deposit is selected from the group consisting of lipomas, fat redistribution syndrome, lipodystrophy, lower eyelid fat herniation, fat deposits associated with cellulite, Dercum's disease, dorsocervical fat, double chin, bra rolls, submental fat, local hyperadiposity, diffused body fat around trunk and arms, and a condition associated with breast reduction.

Such condition may be associated with a rather pathological condition (e.g., lipomas (International Statistical Classification of Diseases and Related Health Problems 10th Revision (ICD-10) of 2015 class D17), Dercum's disease (Lipomatosis dolorosa, ICD-10 class E88.2), lipodystrophy (ICD-10 class E88.1), fat redistribution syndrome, etc.) or be a rather aesthetic concern (e.g., lower eyelid fat herniation, fat deposits associated with cellulite, dorsocervical fat, local hyperadiposity, diffused body fat around trunk and arms, a condition associated with breast reduction, etc.). It will however be understood that, in some cases, pathological conditions and aesthetic concerns are hardly distinguishable.

The pentacyclic triterpenoid compound comprised in the composition of the present invention may be of natural (e.g., of plant, animal, fungal or bacterial origin), of semi-synthetic or of synthetic origin.

In a preferred embodiment, the pentacyclic triterpenoid compound is of plant origin.

In a more preferred embodiment the pentacyclic triterpenoid compound is obtainable from an edible part of a plant.

Such edible part of a plant may exemplarily be a fruit, a fruit peel, a seed, a seed shell, a caulis, a stipe, a cortex, a root, a leaf, a sap, or a brew of a plant or a part of a plant. It will be understood that the plant or part thereof may be processed further such as, e.g., dried, fermented, pulverized, cooked, and/or desiccated, before or while the pentacyclic triterpenoid compound comprised in the composition of the present invention is obtained.

Optionally the pentacyclic triterpenoid compound comprised in the composition of the present invention may be extracted by using a solvent (e.g., water, an aqueous solution and/or one or more organic solvents (e.g., selected from ethanol, methanol, pentane, octanol, etc.)).

In general, the pentacyclic triterpene compound may bear any physicochemical properties.

In a preferred embodiment, the pentacyclic triterpenoid compound is amphiphilic.

Therefore, the pentacyclic triterpenoid compound may comprise a rather hydrophilic (hydrophobic) and a rather lipophilic (hydrophobic) part. Therefore, then, the pentacyclic triterpenoid compound may bear detergent-like properties and may mediate and support the formation and/or stabilization of emulsions between lipophilic and hydrophilic components (e.g., an oil-in-water emulsion).

The pentacyclic triterpenoid compound may be amphiphilic due to one or more partial charges or charges. Preferably, the amphiphilic pentacyclic triterpenoid compound may bear one (i.e. a single) or more charged group(s).In a more preferred embodiment, the pentacyclic triterpenoid compound bears at least one charged group. More preferably, the charged group is an organic charged group.

This charged group may be charged one-fold (i.e., + or -), two-fold (i.e., 2+ or 2-), three-fold (i.e., 3+ or 3-) or more often. Preferably, the charged group is charged one-fold or two-fold, in particular one-fold. The charge may be positively or negatively. In particular, the charge is a one-fold negative charge.

In a particularly preferred embodiment, the pentacyclic triterpenoid compound bears at least one carboxylic group. Particularly preferably, the pentacyclic triterpenoid compound bears one carboxylic group.

The pentacyclic triterpenoid compound may have any structure known for such compound in the art.

In a preferred embodiment, the pentacyclic triterpenoid compound has the following structure according to formula (I) 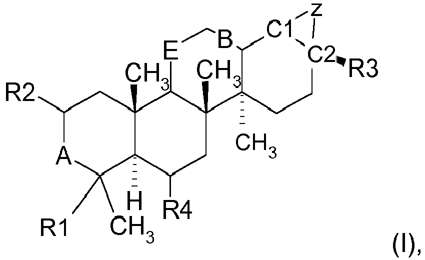 wherein:
R1 is selected from the group consisting of -CH₃, -CH₂OH, and -COO⁻X⁺, wherein X⁺ is a proton or a pharmaceutically acceptable cation;
R2 is hydrogen or -OH;
R3 is selected from the group consisting of -COO⁻X⁺, -CH₃, and -COORa, wherein X⁺ is a proton or a pharmaceutically acceptable cation and wherein Ra is a C₁₋₄-alkyl residue;
R4 is hydrogen or -OH;
C1 and C2 are each a carbon atom wherein the valency of C1 is replenished by hydrogen when the bond to z is a single bond;
z represents a bivalent residue selected from the groups consisting of 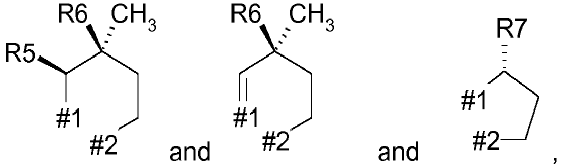 wherein
   #1 represents the binding site to the carbon atom C1 of the remaining structure according to formula (I),
   #2 represents the binding site to the carbon atom C2 of the remaining structure according to formula (I),
   R5 is -CH₃ or hydrogen,
   R6 is -CH₃, hydrogen, or -COO⁻X⁺, and
   R7 is a C₂₋₄ alkenyl residue or a C₁₋₄ alkyl residue, preferably a C₂₋₄ alkenyl residue;
   A is a bivalent residue selected from the groups consisting of
      -CH(OH)-, -CH(OAc)-, -CO-, and -CH₂-; and
      B represents a double or a single bond;
   E represents -CH₂- or -CO-,
in particular wherein one of R1, R3 or R6 is -COO⁻X⁺, wherein X⁺ is a proton or a pharmaceutically acceptable cation.

Herein, the person skilled in the art will note that the carbon atoms C1 and C2 together with the bivalent residue z form a 5- or 6-membered ring, optionally substituted one, two or three times by -CH₃ and substituent(s) R5, R6 and/or R7 as indicated above. Thereby the triterpenoid compound becomes a pentacyclic triterpenoid compound.

The core structure (scaffold) of the pentacyclic triterpenoid structure of the present invention may be an ursane (alpha-Amryn), an oleanane (beta-Amryn) or a lupane ring structure.

In the above structure, X⁺ may be any proton or a pharmaceutically acceptable cation. It is preferably but not necessarily a one-fold positively charged ion. Exemplarily but not limiting, the cation may be selected from the group consisting of an alkali metal (in particular, Na⁺ and/or K⁺), an alkaline earth metal (in particular, Mg²⁺ and/or Ca²⁺ ammonium (NH₄⁺), Fe²⁺, Fe³⁺, Zn²⁺, Sn²⁺, and an organic amine. Preferably, the cation is a proton (H⁺) or an alkali metal (in particular, Na⁺ and/or K⁺).

Preferably, at least one of R1, R3 or R6 is -COO⁻X⁺, wherein X⁺ is a proton or a pharmaceutically acceptable cation.

In a preferred embodiment, herein R7 is -C(=CH₂)-CH₃ and/or A is -CH(OH)- or - CO-. More preferably, one of R1, R3 and R6 is -COO⁻X⁺, wherein X⁺ is a proton or a pharmaceutically acceptable cation. Then, the other residues do preferably not comprise a carboxyl group.

In a highly preferred embodiment, R1 is -CH₃.

Preferably, in the pentacyclic triterpenoid compound according to formula (I), in group z, R7 is -C(=CH₂)-CH₃.

Preferably, in the pentacyclic triterpenoid compound according to formula (I), A is - CH(OH)-, -CH(OAc)- (wherein "Ac" represents an acetyl moiety (-CO-CH₃)) or-CO-.

Preferably, in the pentacyclic triterpenoid compound according to formula (I), B represents a double or a single bond.

In a preferred embodiment, the triterpenoid compound has the following structure according to formula (II) 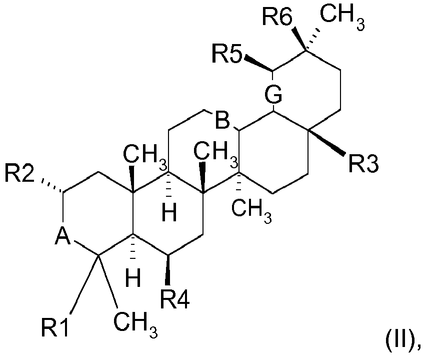 wherein the residues R1 to R6, A and B are defined as in any of claims 1 to 6 and wherein G is a single or a double bond.

In a preferred embodiment, the triterpenoid compound has the following structure according to formula (III) 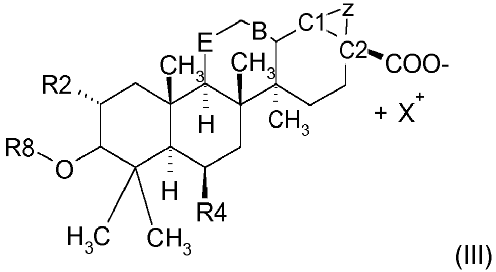 wherein:
C1 and C2 are each a carbon atom;
R2 is hydrogen or -OH;
R4 is hydrogen or -OH, in particular hydrogen;
R5 is -CH₃ or hydrogen,
R6 is -CH₃, hydrogen, or -COO⁻X⁺, and
R8 is hydrogen or -CO-CH₃;
z represents a bivalent residue selected from the group consisting of 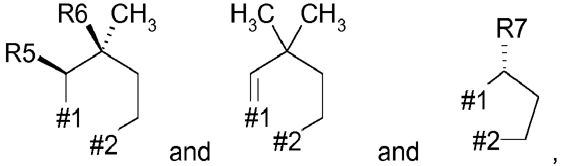 wherein
   #1 represents the binding site to the carbon atom C1 of the remaining structure according to formula III,
   #2 represents the binding site to the carbon atom C2 of the remaining structure according to formula III, and
   B represents a double or a single bond; and
   X⁺ is a proton or a pharmaceutically acceptable cation.

In a particularly preferred embodiment, the triterpenoid compound has a structure according to any of formulae (IV) to (VII): 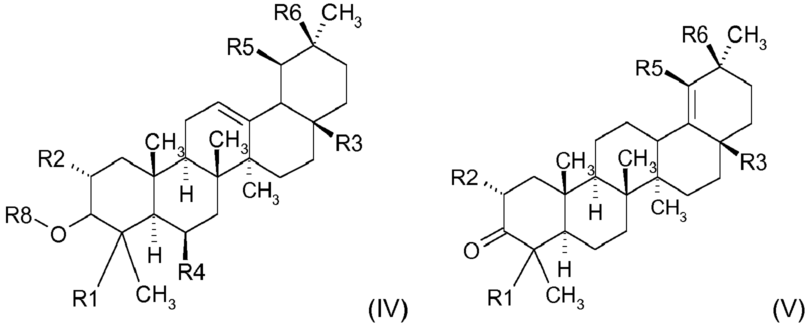 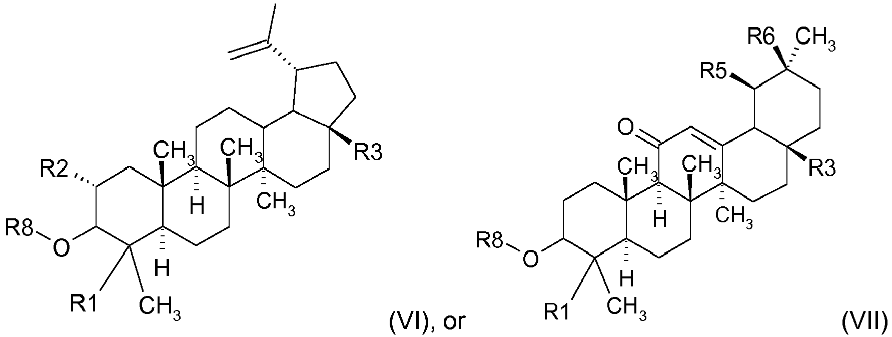 wherein:
R1 is -CH₃, -CH₂-OH, or -COO⁻X⁺, wherein X⁺ is a proton or a pharmaceutically acceptable cation, in particular wherein R1 is -CH₃;
R2 is hydrogen or -OH;
R3 is -COO⁻X⁺ or -CH₃, wherein X⁺ is a proton or a pharmaceutically acceptable cation, in particular wherein R6 is -COO⁻X⁺;
R4 is hydrogen or -OH;
R5 is -CH₃ or hydrogen;
R6 is -CH₃, hydrogen, or -COO⁻X⁺, and
R8 is hydrogen or -CO-CH₃,
wherein one of R1, R3 or R6 is -COO⁻X⁺, wherein X⁺ is a proton or a pharmaceutically acceptable cation.

Even more preferably, the triterpenoid compound has a structure according to any of formulae (VII) to (XII): 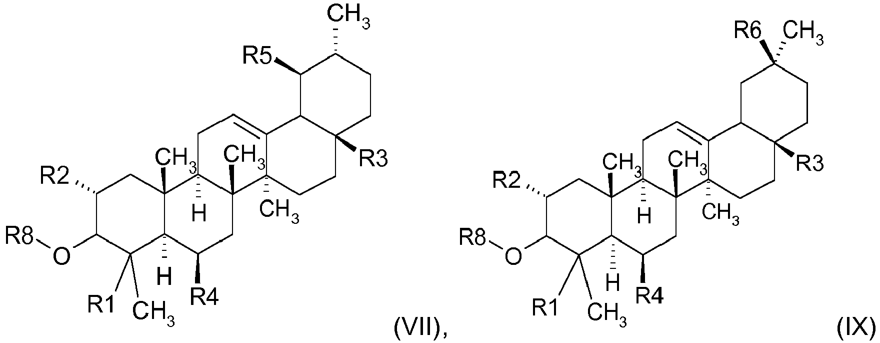 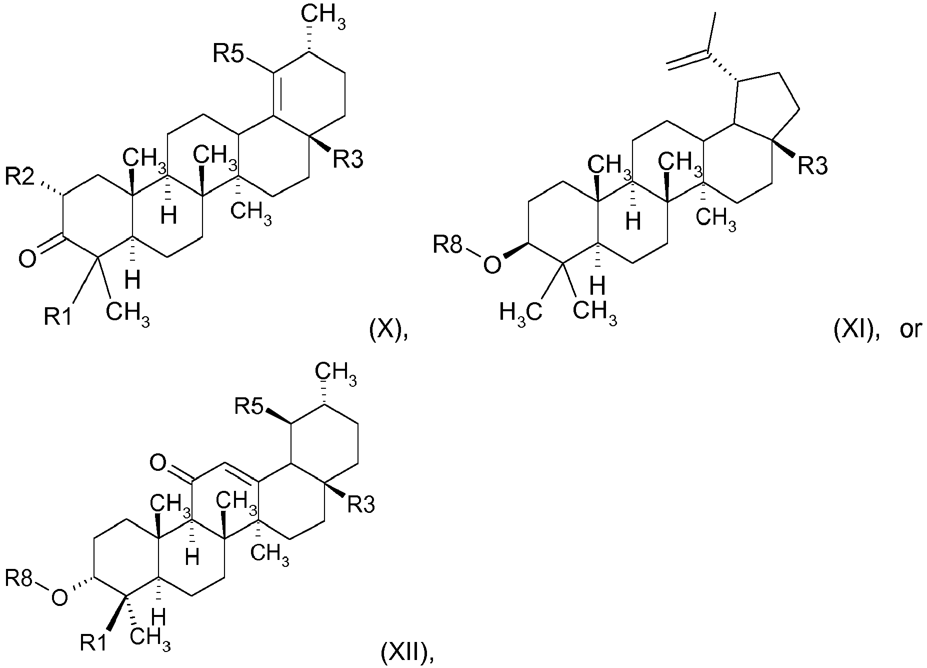 wherein the residues R1-R6 and R7 are defined as above.

As laid out above, it is highly preferred to use pentacyclic triterpenoid compounds obtainable from plants.

Therefore, in a particularly preferred embodiment, the triterpenoid compound is selected from the group consisting of ursolic acid, betulinic acid, moronic acid, oleanolic acid, maslinic acid, asiatic acid, corosolic acid, alpha boswellic acid, beta boswellic acid, acetyl alpha boswellic acid, acetyl beta boswellic acid, acetyl keto alpha boswellic acid, acetyl keto beta boswellic acid, madecassic acid, arjunolic acid, enoxolone, and pharmaceutically acceptable salts thereof.

Ursolic acid is also known as (1S,2R,4aS,6aR,6aS,6bR,8aR,10S,12aR,14bS)-10-hydroxy-1,2,6a,6b,9,9,12a-heptamethyl-2,3,4,5,6,6a,7,8,8a,10,11,12,13,14b-tetradecahydro-1*H*-picene-4a-carboxylic acid, prunol, malol, beta-ursolic acid, NSC4060, CCRIS 7123, TOS-BB-0966, and 3-beta-hydroxyurs-12-en-28-oic acid.

It is exemplarily found and extractable from the peels of a variety of fruits as well as in herbs and spices like rosemary and thyme.

Betulinic acid is also known as (3β)-3-hydroxy-lup-20(29)-en-28-oic acid, 3α-hydroxymethyl-1-isopropenyl-5α,5β,8,8,11α-pentamethyl-icosahydrocyclopenta[a]-chrysenic acid, betulic acid and mairin. It is exemplarily found and extractable from the bark a variety of plants such as, e.g., from the bark of the white birch *Betula pubescens.*

Moronic acid is also known as (4aS,6aR,6aS,6bR,8aS,12aS,14aS)-2,2,6a,6b,9,9,12a-heptamethyl-10-oxo-4,5,6,6a,7,8,8a,11,12,13,14,14a-dodecahydro-3H-picene-4a-carboxylic acid, ambronic acid and 3-oxoolean-18-en-28-oic acid, and oleanolic acid. It is exemplarily found and extractable from *Rhus javanica* and from mistletoe *Phoradendron reichenbachianum.*

Oleanolic acid is also known as (4aS,6aR,6aS,6bR,8aR,10S,12aR,14bS)-10-hydroxy-2,2,6a,6b,9,9,12a-heptamethyl-1,3,4,5,6,6a,7,8,8a,10,11,12,13,14b-tetradecahydropicene-4a-carboxylic acid and Oleanic acid. It is exemplarily found and extractable from various plants such as, e.g., olive oil, American pokeweed *(Phytolacca americana),* garlic, and *Syzygium* species.

Maslinic acid is also known as (4aS,6aR,6aS,6bR,8aR,10R,11R,12aR,14bS)-10,11-dihydroxy-2,2,6a,6b,9,9,12a-heptamethyl-1,3,4,5,6,6a,7,8,8a,10,11,12,13,14b-tetradecahydropicene-4a-carboxylic acid, 2α-hydroxyoleanolic acid and (2α,3β)-2,3-dihydroxyolean-12-en-28-oic acid. It is exemplarily found and extractable from olive oil.

Asiatic acid is also known as (1S,2R,4aS,6aR,6aS,6bR,8aR,9R,10R,11R,12aR, 14bS)-10,11-dihydroxy-9-(hydroxymethyl)-1,2,6a,6b,9,12a-hexamethyl-2,3,4,5,6, 6a,7,8,8a,10,11,12,13,14b-tetradecahydro-1H-picene-4a-carboxylic acid. It is exemplarily found and extractable from *Syzygium claviflorum.*

Corosolic acid is also known as (1S,2R,4aS,6aR,6aS,6bR,8aR,10R, 11R,12aR,14bS)-10,11-Dihydroxy-1,2,6a,6b,9,9,12a-heptamethyl-2,3,4,5,6,6a,7,8, 8a,10,11,12,13,14b-tetradecahydro-1H-picene-4a-carboxylic acid, glucosol; corsolic acid, colosic acid and 2α-hydroxyursolic acid. It is exemplarily found and extractable from *Lagerstroemia speciosa.*

Enoxolone is also known as glycyrrhenic acid (the aglycon of glycyrrhizic acid), glycyrrhetic acid or (2S,4aS,6aS,6bR,8aR,10S,12aS,12bR,14bR)-10-hydroxy-2,4a,6a,6b,9,9,12a-heptamethyl-13-oxo-1,2,3,4,4a,5,6,6a,6b,7,8,8a,9,10,11,12, 12a,12b,13,14b-icosahydropicene-2-carboxylic acid.

Arjunolic acid is also known as 2,3,23-Trihydroxyolean-12-en-28-oic acid or (2α,3β,4α)-2,3,23-Trihydroxy-olean-12-en-28-oic acid. It is exemplarily found and extractable from *Terminalia arjuna, Combretum nelsonii* and/or *Leandra chaeton.*

Madecassic acid is also known as Brahmic acid or (1S,2R,4aS,6aR,6aS, 6bR,8R,8aR,9R,10R,11R,12aR,14bS)-8,10,11-Trihydroxy-9-(hydroxymethyl)-1,2,6a,6b,9,12a-hexamethyl-2,3,4,5,6,6a,7,8,8a,10,11,12,13,14b-tetradecahydro-1 H-picene-4a-carboxylic acid. It is exemplarily found and extractable from *Centella asiatica.*

Beta-boswellic acid is also known as (3α,4β)-3-Hydroxyurs-12-en-23-oic acid, 3α-Hydroxyurs-12-en-24-oic acid. It is exemplarily found and extractable from *Boswellia* species such as, e.g., *Boswellia serrata.*

Alpha boswellic acid is also known as (3α,4β)-3-Hydroxyolean-12-en-23-oic acid, 3α-Hydroxyolean-12-en-24-oic acid. It is exemplarily found and extractable from *Boswellia* species such as, e.g., *Boswellia serrata.*

Likewise, also acetyl beta-boswellic acid, acetyl keto beta-boswellic acid, and acetyl alpha-boswellic acid are each exemplarily found and extractable from *Boswellia* species such as, e.g., *Boswellia serrata.* Further, the respective non-acetylated precursors may also be acetylated synthetically by means of standard methods.

These compounds as such are well-known in the art. They may be obtained from commercial suppliers, isolated from plants or partly or completely synthesized. This is also exemplified in the patent and non-patent documents cited above.

The pentacyclic triterpenoid compound may be a compound having an ursane core structure (alpha-Amryn) selected from the group consisting of ursolic acid, beta boswellic acid, corosolic acid, asiatic acid, madecassic acid, acetyl beta boswellic acid, and acetyl keto beta boswellic acid, and pharmaceutically acceptable salts thereof.

Alternatively, the pentacyclic triterpenoid compound may be a compound having an oleanane core structure (beta-Amryn) selected from the group consisting of maslinic acid, oleanolic acid, moronic acid, arjunolic acid, alpha boswellic acid, acetyl alpha boswellic acid, acetyl keto alpha boswellic acid, and enoxolone, and pharmaceutically acceptable salts thereof.

Alternatively, the pentacyclic triterpenoid compound may be a compound having a lupan core structure such as betulinic acid or a pharmaceutically acceptable salt thereof.

More preferably, the pentacyclic triterpenoid compound is selected from the group consisting of ursolic acid, betulinic acid, moronic acid, oleanolic acid, maslinic acid, asiatic acid, corosolic acid, alpha boswellic acid, and beta boswellic acid and pharmaceutically acceptable salts thereof.

The pentacyclic triterpenoid compound may be selected from the group consisting of ursolic acid, betulinic acid, moronic acid, oleanolic acid, maslinic acid, asiatic acid, corosolic acid and pharmaceutically acceptable salts thereof.

The pentacyclic triterpenoid compound may be selected from the group consisting of ursolic acid, betulinic acid and maslinic acid and pharmaceutically acceptable salts thereof.

The pentacyclic triterpenoid compound may exemplarily be maslinic acid or a pharmaceutically acceptable salt thereof.

The composition may be any pharmaceutically acceptable composition suitable for being injected into a fat deposit comprising the compound of the present invention. In a preferred embodiment, in the composition, the composition is a buffered or unbuffered (in particular buffered), isotonic or hypertonic (in particular isotonic) aqueous or non-aqueous composition comprising the compound of the present invention or pharmaceutically acceptable salt thereof in an amount of 0.01 to 10 % by weight, more preferably in an amount of 0.05 to 5 % by weight, even more preferably in an amount of 0.07 to 3 % by weight, even more preferably in an amount of 0.1 to 2%, even more preferably in an amount of 0.1 to 1.5 %, in particular 0.1 to 1.0 % by weight.

The composition of the present invention may in principle comprise the pentacyclic triterpenoid compound and the at least one further component C in any ratios.

Preferably, the composition consists of:
(A) 0.1 to 5 % (w/w) of one or more pentacyclic triterpenoid compounds or pharmaceutically acceptable salts thereof for use as laid out above or one or more pharmaceutically acceptable amino acid and/or acyl conjugates thereof;
(B) 45 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 10 % (w/w) of one or more pharmaceutically acceptable detergents; and
(D) 0 to 40 % (w/w) of one or more pharmaceutically acceptable additives.

As used herein in the context of the contents of the components (A), (B), (C) and/or (D) in the composition, the percentage ranges refer to the contents of the respective component(s) in the composition as a whole (comprising or consisting of the components (A)-(D)).

More preferably, the composition consists of:
(A) 0.1 to 3 % (w/w) of one or more pentacyclic triterpenoid compounds or pharmaceutically acceptable salts thereof for use as laid out above or one or more pharmaceutically acceptable amino acid and/or acyl conjugates thereof;
(B) 67 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 10% (w/w) of one or more pharmaceutically acceptable detergents; and
(D) 0 to 20 % (w/w) of one or more pharmaceutically acceptable additives.

Even more preferably, the composition consists of:
(A) 0.1 to 2 % (w/w) of one or more pentacyclic triterpenoid compounds or pharmaceutically acceptable salts thereof for use as laid out above or one or more pharmaceutically acceptable amino acid and/or acyl conjugates thereof;
(B) 73 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 5% (w/w) of one or more pharmaceutically acceptable detergents; and
(D) 0 to 20 % (w/w) of one or more pharmaceutically acceptable additives.

In an even more preferred embodiment, the composition consists of:
(A) 0.1 to 2 % (w/w) of one or more pentacyclic triterpenoid compounds or pharmaceutically acceptable salts thereof for use as laid out above or one or more pharmaceutically acceptable amino acid and/or acyl conjugates thereof;
(B) 81 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

In an even more preferred embodiment, the composition consists of:
(A) 0.1 to 2 % (w/w) of one or more pentacyclic triterpenoid compounds or pharmaceutically acceptable salts thereof for use as laid out above or one or more pharmaceutically acceptable amino acid and/or acyl conjugates thereof;
(B) 91 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

In an even more preferred embodiment, the composition consists of:
(A) 0.1 to 1.5 % (w/w) of one or more pentacyclic triterpenoid compounds or pharmaceutically acceptable salts thereof for use as laid out above or one or more pharmaceutically acceptable amino acid and/or acyl conjugates thereof;
(B) 91.5 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

Due to the fact that the pentacyclic triterpenoid compound comprised in the composition of the present invention is intended to be injected into the undesired local subcutaneous fat deposit in a mammal, the composition is preferably administered to the mammal in liquid or viscous form. Therefore, the composition is preferably liquid or pasty, in particular pasty. Particularly preferably, the composition is administered to the mammal in liquid form. Therefore, the composition is particularly preferably liquid. It will however be understood that the composition may optionally also be stored and/or delivered in solid state and then be dissolved, suspended or emulsified just prior to use. This may increase shelf life thereof.

A pharmaceutically acceptable carrier according the present invention may be any additive that is pharmaceutically acceptable, therefore, any additive that is nontoxic to the mammal. As the composition is preferably liquid or pasty, in particular liquid, the pharmaceutically acceptable carrier preferably comprises or consists of one or more solvents such as, e.g., water, an aqueous buffer (e.g., a saline or phosphate buffered saline), dimethyl sulfoxide (DMSO), ethanol, vegetable oil, paraffin oil or combinations thereof.

The composition of the present invention comprises at least one pentacyclic triterpenoid compound. Optionally, the composition may also comprise more that one pentacyclic triterpenoid compound comprised in the composition of the present invention such as the combination of two, three, four, five or even more pentacyclic triterpenoid compounds of the present invention.

Furthermore, a pharmaceutically acceptable carrier may optionally further contain one or more detergent(s) such as, e.g., sodium lauryl sulfate (SLS)/ sodium doceyl sulfate (SDS).

Furthermore, a pharmaceutically acceptable carrier may optionally further contain one or more pharmaceutically acceptable additives. Such pharmaceutically acceptable additives may exemplarily be selected from the group consisting of one or more coloring agent(s) (e.g., TiO₂, food coloring), one or more vitamin(s), one or more salt(s) (e.g., sodium, potassium, magnesium, calcium, and/or zinc salts), one or more humectant(s) (e.g., sorbitol, glycerol, mannitol, propylene glycol, polydextrose), one or more enzyme(s), one or more preserving agent(s) (e.g., benzoic acid, methylparabene), one or more texturing agent(s) (e.g., carboxymethyl cellulose (CMC), polyethylene glycol (PEG), sorbitol), one or more emulsifier(s), one or more separating agent(s), one or more antioxidant(s), one or more herbal and plant extract(s), one or more stabilizing agent(s), one or more polymer(s) (e.g., hydroxypropyl methacrylamide (HPMA), polyethylene imine (PEI), carboxymethyl cellulose (CMC), polyethylene glycol (PEG)), one or more uptake mediator(s) (e.g., polyethylene imine (PEI), one or more solvents (e.g., dimethyl sulfoxide (DMSO)), a cell-penetrating peptide (CPP), a protein transduction domain (PTD), an antimicrobial peptide, etc.) one or more antibody/antibodies, one or more counterstain dye(s) (e.g., fluorescein, fluorescein derivatives, Cy dyes, an Alexa Fluor dye(s), S dye(s), rhodamine, quantum dot(s), etc.), and one or more homeopathic ingredient(s).

The composition may or may not comprise one or more natural bile acids (e.g., deoxycholate (DC), chenodeoxycholis acid (CDCA), ursodeoxycholate (UDCA) and/or lithocholic acid (LCA)) and/or phosphatidylcholine (PC). Preferably, the composition of the present invention is free or at least essentially free of deoxycholate (DC), chenodeoxycholis acid (CDCA), ursodeoxycholate (UDCA), lithocholic acid (LCA) and phosphatidylcholine (PC). Should one or more of the aforementioned agent be present in the composition, this agent can be used in lower amounts due to the presence of the pentacyclic triterpenoid compound comprised in the composition of the present invention.

The present invention also relates to a method of treating or preventing a condition associated with an undesired local subcutaneous fat deposit in a mammal, wherein a sufficient amount of a composition according to the present invention comprising or consisting of:
(A) 0.1 to 5 % (w/w) of one or more pentacyclic triterpenoid compounds or pharmaceutically acceptable salts thereof for use according to the present invention, one or more pharmaceutically acceptable prodrugs thereof such as amino acid and/or acyl conjugates thereof, solvates thereof, and/or complexes thereof;
(B) 85 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 5 % (w/w) of one or more detergents; and
(D) 0 to 5 % (w/w) of one or more pharmaceutically acceptable additives.

In this context, it will be understood that all definitions provided above apply *mutatis mutandis* to this aspect of the present invention.

The pentacyclic triterpenoid compounds may be embedded into a pharmaceutically acceptable structure that is suitable for injections such as, e.g., a dispersion, in particular an emulsion (e.g., an oil-in-water (O/W) emulsion, a water-in-oil (W/O) emulsion, a water-in-oil-in water (W/O/W) emulsion, or an oil-in-water-oil emulsion (O/W/O), in particular a micelle or a liposome structure), a suspension of the triterpenoid compound itself, a suspension of beads (e.g., beads comprising said compound in the interior and/or in a shell structure and/or attached to the bead surface, wherein the beads may exemplarily be microbeads and/or nanobeads), a liquid or semi-solid (i.e., gel-like) solution/suspension wherein said compound is dissolved/suspended, a liquid or semi-liquid composition comprising fibers (e.g., a hydrogel) in which said compound is incorporated and/or attached to, soluble, partly soluble or insoluble biocompatible polymer strands to which the compound is attached to (e.g., hydroxypropyl methacrylamide (HPMA), polyethylene imine (PEI), carboxymethyl cellulose (CMC), polyethylene glycol (PEG), polylactic acid (PLA), polyethylene imine (PEI), polypeptides, polysaccharides (e., dextran), or a combination of two or more thereof), or a combination of two or more thereof. Examples for a variety of such pharmaceutical formulations are provided in US 2005/0196416 and US 2004/0180082.

In a preferred embodiment, the pentacyclic triterpenoid compounds are embedded in a liposome structure.

As indicated above, the present invention also refers to cosmetic uses of the pentacyclic triterpenoid compound comprised in the composition of the present invention.

Therefore, in a further aspect, the present invention refers to a method for aesthetic medical or cosmetic reduction of an undesired local subcutaneous fat deposit, comprising the step of locally injecting a sufficient amount of a composition as laid out above, in particular wherein said method is used for body contouring, in particular via adipocytolysis.

In this context, it will be understood that all definitions provided above apply *mutatis mutandis* to this aspect of the present invention. The aesthetic medical and cosmetic use can be any use associated with an undesired local subcutaneous fat deposit known in the art. In a particularly preferred embodiment, the method according to the present invention is used for body contouring.

Therefore, the outer shape and appearance of the mammal's body may be changed and potentially improved in that the one or more undesired local subcutaneous fat deposit(s) are (optically) removed or at least reduced in size.

A still further aspect of the present invention relates to the use of a pentacyclic triterpenoid compound or a pharmaceutically acceptable salt thereof for body contouring by means of injection lipolysis.

In this context, it will be understood that all definitions provided above apply *mutatis mutandis* to this aspect of the present invention.

In a preferred embodiment the pentacyclic triterpenoid compound is defined as laid out above and is optionally comprised in a composition according to the present invention.

The invention is further explained by the following examples and claims.

### Brief Description of the Figures

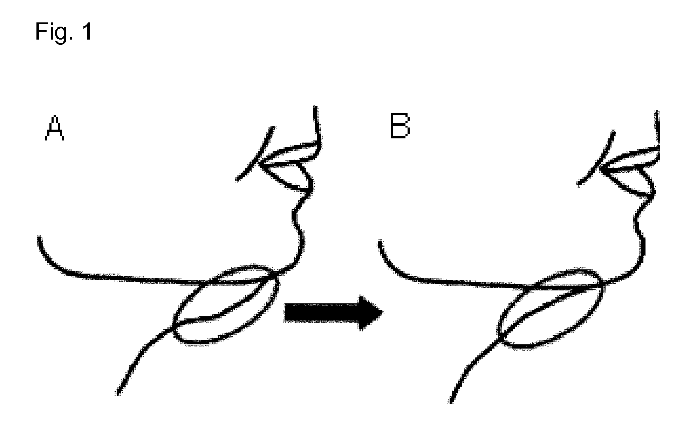

Figure 1 schematically shows an example of the technical effect of the composition of the present invention, i.e., the treatment of a double chin. A: before the treatment, B: after the treatment.

### Examples

### In vitro use of pentacyclic triterpenoid compounds

Summarized, maslinic acid serving as an example for pentacyclic triterpenoid compounds provides surprisingly good results in acting against adipocytes. In comparison to deoxycholate, maslinic acid bears a superior adipocytolytic effect on human adipocytes. At a concentration of 50 µM of maslinic acid, the viability of adipocytes decreases to approximately 50% whereas a concentration as high as 200 µM (i.e., the 4fold concentration) of deoxycholate is required to achieve such 50%-decrease in viability. Likewise, also LDH release (indicative for membrane damage) from the adipocytes is higher when using maslinic acid instead of deoxycholate, but far not as high as 4fold.

Therefore, reducing the vitality of adipocytes by means of maslinic acid notably and unexpectedly follows a milder mechanism of action compared to the employment of deoxycholate. Maslinic acid further reveals as being pro-apoptotic. In brief, maslinic acid is more effective and has the potential to provoke lower side effects compared to deoxycholate.

### Results of the Viability Testing

The following experimental setup was used for determination of adipocytolytic potential of test compounds with preadipocytes and mature adipocytes:
Primary human preadipocytes (PromoCell) were seeded in Preadipocyte Growth Medium at 45000 cells/cm² to allow attachment overnight. Cells were cultured at 37°C and 5% CO₂ until postconfluence. Differentiation was started with incubation of cells in Differentiation Medium for 7 days followed by incubation in Nutrition medium for further 14 days. After differentiation process, mature adipocytes were incubated with indicated concentration of indicated triterpenes.

6 replicates of each test compound concentration were tested. Each plate included a negative control and a compound interference control (for all concentrations, duplicates).

Cell viability as surrogate for adipocytolytic potential of compounds were analysed by alamar blue assay. In brief, 20µl of a 0,1mg/ml Resazurin sodium salt (Sigma; R7017-1 G) solution was added to each well 48 hrs after compound incubation. The cells were incubated for 90 min at 37°C and 5% CO₂ and the resulting conversion of resazurin to resorufin was measured by a fluorescence plate reader at 590 nm. Vehicle controls were set as 100%. IC50-values were analysed using SigmaBlotV13.

For analysing the potential of compounds to induce apoptosis the Caspase-Glo® 3/7 Assay (Promega) was used as recommended by the supplier. In brief, 100µl Caspase Reagent were added to 100µl cell culture medium and cells were incubated at 37°C and 5% CO₂ for 1 hr. Luminescence signal was measured with a SynergyH4 plate reader. Vehicle controls were used to define basal apoptosis as 100%.

**Table 1 Impact on viability of adipocytes after 48h incubation with indicated compound in increasing concentration**

| | Viability Adipocytes (%) | | | | | |
|---|---|---|---|---|---|---|
| Conc (µM) | Deoxycholic acid | Betulinic acid | Corosolic acid | Maslinic acid | Oleanolic acid | Ursolic acid |
| 10 | 106.98 | 103.70 | 92.93 | 96.86 | 114.40 | 92.36 |
| 30 | - | 85.61 | 60.52 | 76.70 | 110.06 | 28.93 |
| 50 | 95.61 | 70.72 | 45.31 | 47.08 | 116.79 | 14.87 |
| 100 | 94.30 | 32.28 | 6.06 | 4.86 | 32.36 | 13.36 |
| 300 | 22.06 | 4.90 | 0.47 | 0.30 | 15.79 | 10.34 |

**Table 2 Impact on viability of pre-adipocytes after 48h incubation with indicated compound in increasing concentration**

| | Viability Preadipocytes (%) | | | | | |
|---|---|---|---|---|---|---|
| Conc (µM) | Deoxycholic acid | Betulinic acid | Corosolic acid | Maslinic acid | Oleanolic acid | Ursolic acid |
| 10 | 96.22 | 81.77 | 94.66 | 96.37 | - | 74.78 |
| 30 | 92.76 | 48.53 | 68.63 | 76.00 | 79.11 | 12.71 |
| 50 | 95.61 | 45.12 | 20.36 | 45.60 | 83.43 | 0.32 |
| 100 | 98.03 | 17.49 | 0.00 | 2.42 | 53.33 | 0.00 |
| 300 | 26.08 | 0.45 | 0.00 | 0.00 | 0.00 | 0.00 |

**Table 3 IC50-values of tested triterpenes and deoxycholic acid**

| Compound | IC50 [µM] | IC50 [µM] |
|---|---|---|
| | Adipocytes | Pre-ad ipocytes |
| Corosolic acid | 45.03 | 43.07 |
| Maslinic acid | 53.43 | 54.26 |
| Ursolic acid | 24.40 | 20.05 |
| Betulinic acid | 72.00 | 35.50 |
| Oleanolic acid | 92.80 | 124.70 |
| Deoxycholic acid | 243.60 | 232.40 |

All tested triterpenes show a dose dependent reduction of viability with a lower IC50 value compared to deoxycholic acid both on preadipocytes and adipocytes. The differences range from 2.5fold for Oleanolic acid to about 10fold for Ursolic acid. Taken together, the triterpenes demonstrate 2.5-10 times higher adipocytolytic effect compared to deoxycholic acid.

**Table 4 Impact on apoptosis induction of adipocytes after 6hrs and 48hrs with indicated compound concentrations of maslinic acid**

| | Apoptosis Adipocytes (%) | |
|---|---|---|
| conc (µM) | Maslinic acid 6 hrs | Maslinic acid 48hrs |
| 30 | 97.17 | 121.70 |
| 50 | 126.43 | 116.95 |
| 100 | 662.48 | 304.15 |
| 200 | - | 378.25 |
| 300 | 398.94 | 238.76 |

After short time incubation concentrations from 50 µM on show clear induction of apoptosis with max. (6.6fold over basal levels) at 100 µM after 6 hrs. With extended incubation time apoptosis induction is also measurable at lower concentrations.

In summary data show a clear pro-apoptotic capacity of maslinic acid.

## Claims

1. A composition comprising a pentacyclic triterpenoid compound or a pharmaceutically acceptable salt thereof, and at least one further component C for use in a method for local treating or preventing a condition associated with an undesired local subcutaneous fat deposit in a mammal, wherein said compound is injected into said local subcutaneous fat deposit.

2. The composition for use according to claim 1, wherein injecting said composition into the local subcutaneous fat deposit is for reducing the size of said local subcutaneous fat deposit, in particular for body contouring.

3. The composition for use according to claim 1 or 2, wherein the condition associated with an undesired local subcutaneous fat deposit is selected from the group consisting of lipomas, fat redistribution syndrome, lipodystrophy, lower eyelid fat herniation, fat deposits associated with cellulite, Dercum's disease, dorsocervical fat, double chin, bra rolls, submental fat, local hyperadiposity, diffused body fat around trunk and arms, and a condition associated with breast reduction.

4. The composition for use according to any of claims 1 to 3, wherein the triterpenoid compound is of plant origin, in particular obtainable from an edible part of a plant.

5. The composition for use according to any of claims 1 to 4, wherein the triterpenoid compound is amphiphilic and bears at least one charged group, in particular one carboxylic group.

6. The composition for use according to any of claim 1 to 5, wherein the triterpenoid compound has the following structure according to formula (I) 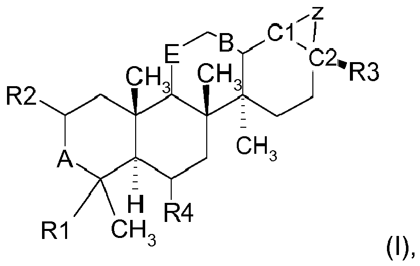 wherein:
R1 is selected from the group consisting of -CH₃, -CH₂OH, and -COO⁻X⁺, wherein X⁺ is a proton or a pharmaceutically acceptable cation;
R2 is hydrogen or -OH;
R3 is selected from the group consisting of -COO⁻X⁺, -CH₃, and -COORa,
wherein X⁺ is a proton or a pharmaceutically acceptable cation and wherein Ra is a C₁₋₄-alkyl residue;
R4 is hydrogen or -OH;
C1 and C2 are each a carbon atom, wherein the valency of C1 is replenished by hydrogen when the bond to z is a single bond;
z represents a bivalent residue selected from the groups consisting of 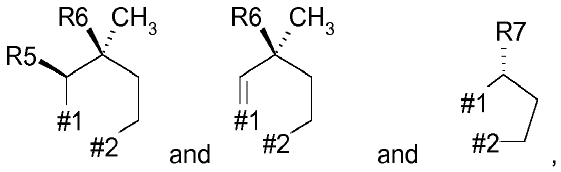 wherein
#1 represents the binding site to the carbon atom C1 of the remaining structure according to formula (I),
#2 represents the binding site to the carbon atom C2 of the remaining structure according to formula (I),
R5 is -CH₃ or hydrogen,
R6 is -CH₃, hydrogen, or -COO⁻X⁺, and
R7 is a C₂₋₄ alkenyl residue or a C₁₋₄ alkyl residue;
A is a bivalent residue selected from the groups consisting of
-CH(OH)-, -CH(OAc)-, -CO-, and -CH₂-; and
B represents a double or a single bond;
E represents -CH₂- or -CO-,
in particular wherein one of R1, R3 or R6 is -COO⁻X⁺, wherein X⁺ is a proton or a pharmaceutically acceptable cation.

7. The composition for use according to claim 6, wherein in formula (I) R7 is-C(=CH₂)-CH₃ and/or A is -CH(OH)- or -CO-.

8. The composition for use according to any of claims 1 to 7, wherein the triterpenoid compound has the following structure according to formula (II) 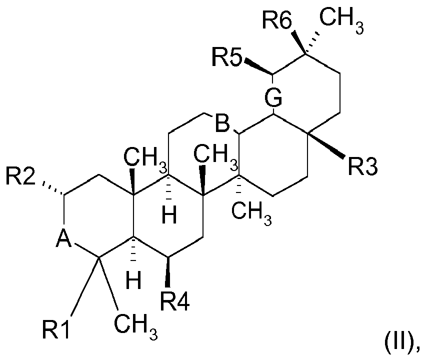 wherein the residues R1 to R6, A and B are defined as in any of claims 1 to 6 and wherein G is a single or a double bond.

9. The composition for use according to any of claims 1 to 7, wherein the triterpenoid compound has the following structure according to formula (III) 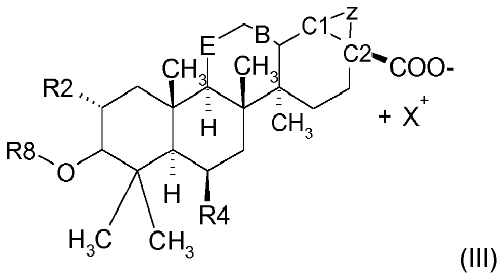 wherein:
C1 and C2 are each a carbon atom;
R2 is hydrogen or -OH;
R4 is hydrogen or -OH, in particular hydrogen;
R5 is -CH₃ or hydrogen,
R6 is -CH₃, hydrogen, or -COO⁻X⁺, and
R8 is hydrogen or -CO-CH₃;
z represents a bivalent residue selected from the group consisting of 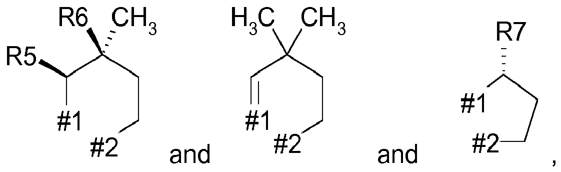 wherein
#1 represents the binding site to the carbon atom C1 of the remaining structure according to formula III,
#2 represents the binding site to the carbon atom C2 of the remaining structure according to formula III, and
B represents a double or a single bond; and
X⁺ is a proton or a pharmaceutically acceptable cation.

10. The composition for use according to any of claims 1 to 7, wherein the triterpenoid compound has a structure according to any of formulae (IV) to (VII): 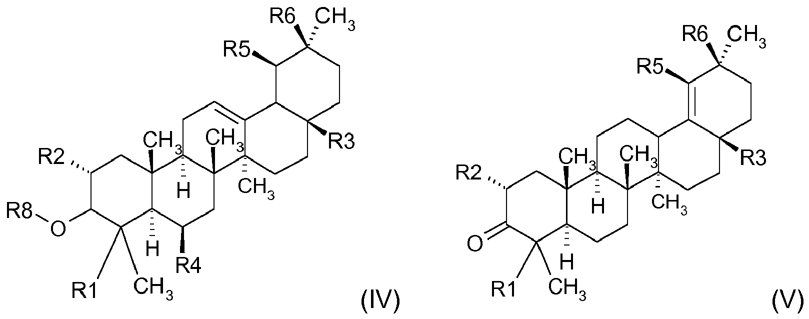 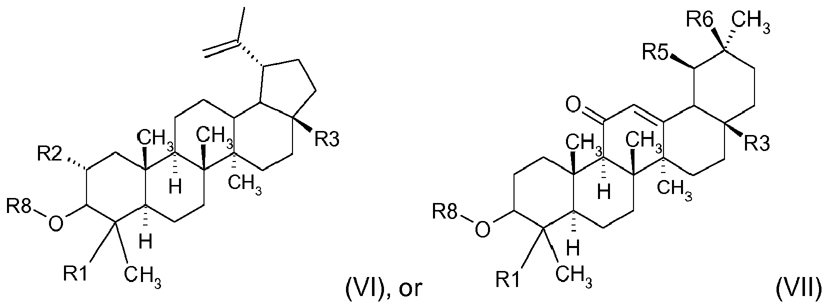 wherein:
R1 is -CH₃, -CH₂-OH, or -COO⁻X⁺, wherein X⁺ is a proton or a pharmaceutically acceptable cation, in particular wherein R1 is -CH₃;
R2 is hydrogen or -OH;
R3 is -COO⁻X⁺ or -CH₃, wherein X⁺ is a proton or a pharmaceutically acceptable cation, in particular wherein R6 is -COO⁻X⁺;
R4 is hydrogen or -OH;
R5 is -CH₃ or hydrogen;
R6 is -CH₃, hydrogen, or -COO⁻X⁺, and
R8 is hydrogen or -CO-CH₃,
wherein one of R1, R3 or R6 is -COO⁻X⁺, wherein X⁺ is a proton or a pharmaceutically acceptable cation.

11. The composition for use according to any of claims 1 to 10, wherein the triterpenoid compound is selected from the group consisting of ursolic acid, betulinic acid, moronic acid, oleanolic acid, maslinic acid, asiatic acid, corosolic acid, alpha boswellic acid, beta boswellic acid, acetyl alpha boswellic acid, acetyl beta boswellic acid, acetyl keto alpha boswellic acid, acetyl keto beta boswellic acid, madecassic acid, arjunolic acid, enoxolone, and pharmaceutically acceptable salts thereof.

12. The composition for use according to any of claims 1 to 11, wherein said composition consists of:
(A) 0.1 to 2 % (w/w) of one or more pentacyclic triterpenoid compounds or pharmaceutically acceptable salts thereof for use according to any of claims 1 to 11 or one or more pharmaceutically acceptable amino acid and/or acyl conjugates thereof;
(B) 91 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

13. The composition for use according to any of claims 1 to 12, wherein the pentacyclic triterpenoid compounds are embedded in a liposome structure.

14. A method for cosmetic reduction of an undesired local subcutaneous fat deposit, comprising the step of locally injecting a sufficient amount of a composition according to any of claims 1 to 13, in particular wherein said method is used for body contouring, in particular via adipocytolysis.

15. Use of a pentacyclic triterpenoid compound or a pharmaceutically acceptable salt thereof for body contouring by means of adipocytolysis, in particular wherein said compound is defined as in any of claims 1 to 13.
